# EUROPEAN PATENT SPECIFICATION

(11) **EP 2 835 430 B1**
(45) Date of publication and mention of the grant of the patent: **30.03.2016**
(21) Application number: 14177653.4
(22) Date of filing: 18.07.2014
(51) Int. Cl.: C14B 17/00, C14B 1/28, C14B 17/06, G01N 33/44, G07C 3/14

(54) **System for classifying hides according to quality**
System zur Klassifizierung von Häuten nach Qualität
Système permettant de classer des peaux selon la qualité

(30) Priority: 06.08.2013 IT VI20130206
(43) Date of publication of application: 11.02.2015
(73) Proprietor: Feltre Srl, 36050 Montorso Vicentino (VI) (IT)
(72) Inventor: Feltre, Domenico, 37030 Ronca' (VR) (IT)
(74) Representative: Bonini, Ercole

(56) References cited:
- EP-A1- 0 023 812
- EP-A1- 1 772 524
- WO-A2-2008/022248
- US-A- 4 199 255

## Description

The invention concerns a system for classifying hides according to their quality.

It is known that at the end of the processing cycles hides are subjected to a series of checks intended to sort them into groups of homogeneous quality.

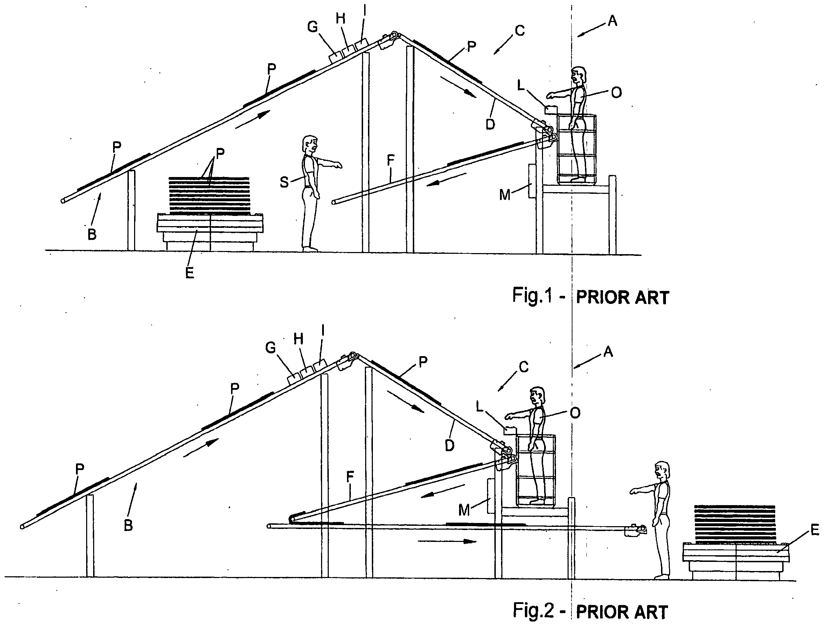

For this purpose, systems of the type shown in Figure 1 or in Figure 2, where they are indicated by A, are used, in which the hides P to be controlled and classified advance, through a feeding line B, towards a control station C, in which they transit resting on an inclined control surface D in order to be examined by an operator O.

The hides P move along the feeding line B that is provided with automatic means suited to measure their physical characteristics like, for example, their surface area G, their weight H, their thickness I and other characteristics. Furthermore, the operator O who checks the advancing hide P, which is spread on the control surface D, can use a manual unit L by means of which, at the end of the check and based on his/her experience, he/she assigns the hide P a quality code selected among a predetermined series of codes.

All these data are stored in a classification unit schematically indicated by M and comprising electronic processing and control means that classify the quality of the hide by processing the data according to a predefined algorithm. At the end of the control process, an unloading device S collects the hide from the outlet surface F positioned downstream of the control station C and stacks it on one of many available pallets, only one of which, indicated by E, is shown in Figures 1 and 2, wherein each one of said pallets corresponds to the quality classification made by the classification unit M.

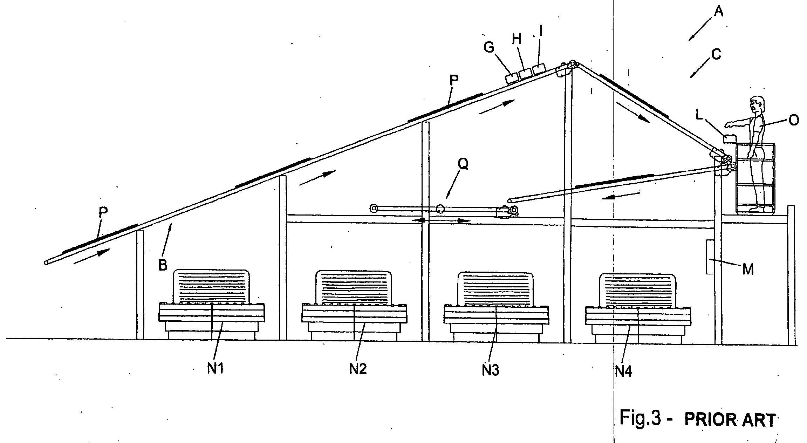

The systems of known type are preferably carried out according to the configuration shown in Figure 3, in which the classification unit M controls the operation of power and moving means indicated as a whole by Q and arranged downstream of the control station C in order to stack the controlled hides on corresponding accumulation pallets N1, N2, N3, N4 arranged in corresponding accumulation stations, wherein each pallet is prepared so that it can accommodate classified hides of the same quality or hides in a predefined quality mix, according to the needs of the final user of the hides.

A system for classifying hides according to their quality of the type just described above is also known from the patent document EP 0 023 812 A1 that describes a system comprising a hide conveyor and a unit for classifying hides based on their quality.

All the known systems for hide classification, in all their different embodiments of which Figures from 1 to 3 show only some examples, however pose a series of recognized drawbacks and limitations.

A first limitation is constituted by the fact that when said systems are arranged downstream of the modern high-output finishing machines the only operator present in the only control station does not have enough time to carry out an accurate check on the hide, especially when he/she has to classify high-quality hides.

In these cases the operator is forced to reduce the advance speed of the hides, with the drawback that the hides leaving the processing lines accumulate upstream of the system.

Another drawback is constituted by the fact that when, due to different reasons, it is necessary to stop a hide in the control station for a prolonged time, all the control system must be stopped.

Another and not less important drawback is represented by the fact that when the system is used to control hides of different type and quality that require very different control times, the overall output of the system is reduced and conditioned by those hides that require longer control times.

The present invention intends to overcome all the drawbacks listed above.

In particular, it is one of the objects of the invention to provide a hide quality classification system that is always capable of adapting its hide classification capacity to the speed at which the hides leave the processing lines located upstream, independently of the speed.

It is another object of the invention to provide a system that makes it possible to prolong the time during which a hide remains stationary in order to be subjected to the check, without however stopping the entire control line.

It is another and not less important object of the invention to provide a classification system that is flexible and suited to control hides requiring different control times.

It is another and not less important object of the invention to provide a system that allows higher outputs than the known systems.

The objects described above are achieved by a hide quality classification system according to the main claim, to which reference is made herein. Advantageously, the system that is the subject of the invention is more flexible than the known systems and therefore more adaptable to the needs of the user.

Still advantageously, the classification system that is the subject of the invention allows considerable efficiency increases in terms of number of hides checked per hour.

The objects and advantages described above will be highlighted in greater detail here below, in the description of a preferred but not exclusive embodiment of the invention that is provided by way of non- limiting example with reference to the attached drawings, wherein:
- Figures from 1 to 3 show three different embodiments of systems of the known art;
- Figure 4 shows an embodiment of the classification system that is the subject of the invention;
- Figure 4a shows a detail of Figure 4;
- Figure 5 shows the system of Figure 4 in a different operating position;
- Figure 5a shows a detail of Figure 5;
- Figures 6, 6a; 7, 7a; 8, 8a; 9, 9a show the ends of the mutually converging transport and control surfaces of the system of the invention, in different operating positions;
- Figure 10 shows a variant embodiment of the classification system that is the subject of the invention;
- Figure 11 shows another variant embodiment of the classification system that is the subject of the invention.

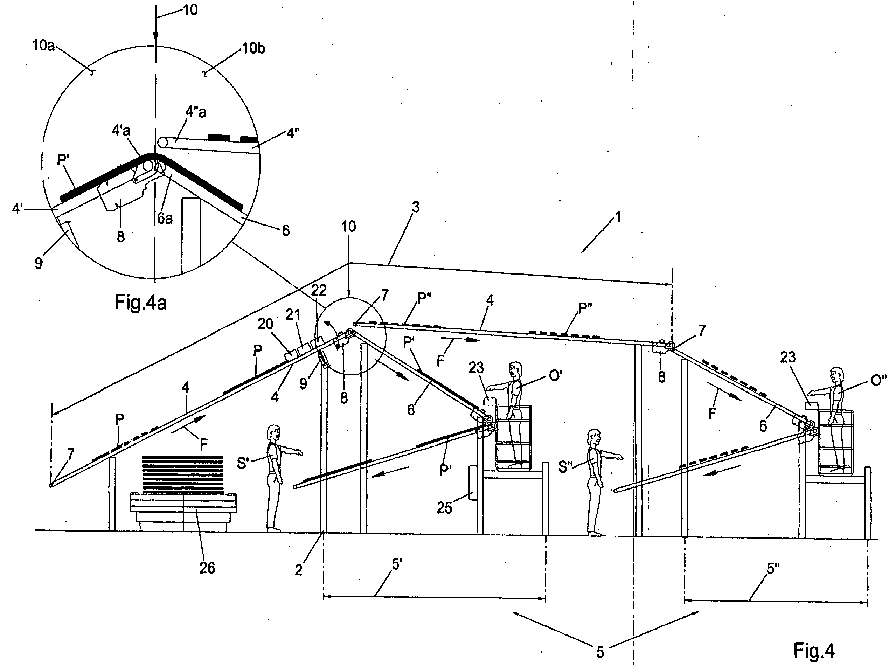

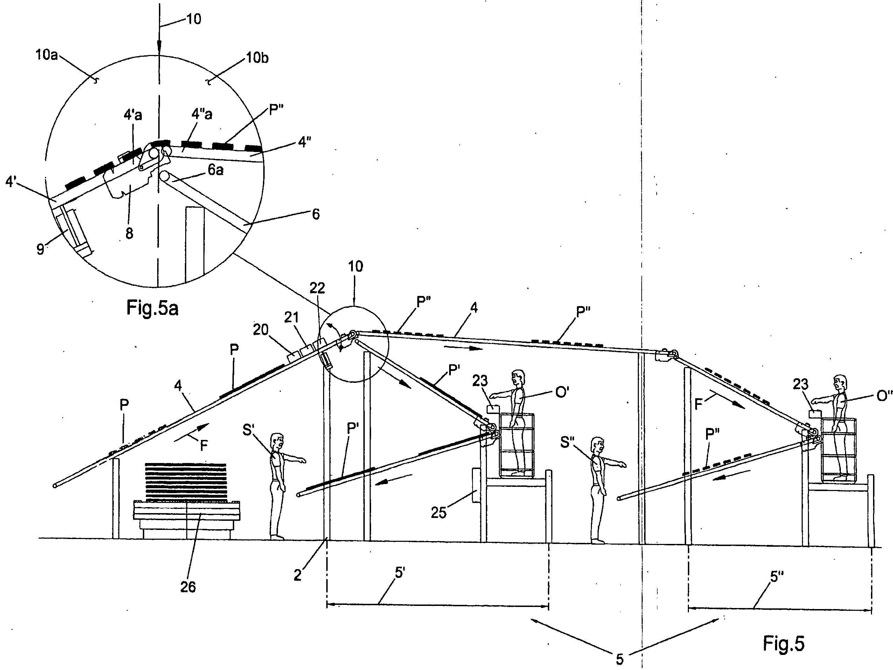

The system of the invention, indicated as a whole by **1,** is shown in Figures 4 and 5 in two different operating positions.

It can be observed that it comprises a frame **2** supporting a feeding line **3** that comprises a plurality of distinct transport surfaces **4** arranged one after the other in order to make said hides **P** move forward according to the direction indicated by the arrow **F** until they reach a plurality of control stations **5.**

In the embodiment of the invention described herein, the control stations **5** are two and comprise a first control station **5'** and a second control station **5".**

It is clear, however, as will be explained in greater detail below, that there can be any number of control stations.

It can be observed that each one of the control stations **5** comprises a control surface **6** that is preferably but not necessarily inclined in such a way as to allow the operator **O**, who is present in the control station, to analyse the hide and assess its quality.

The control surface **6** may also comprise several plane and/or inclined control surfaces, arranged one after the other.

It can be observed, in particular, that the control stations **5** are connected in parallel to the feeding line **3** through a sorting station **10** that serves to selectively transfer the hides **P** between the transport surfaces **4** and the control surfaces **6** so as to make them reach the predefined control station **5'** or **5**".

Regarding the transport surfaces **4,** each one of them comprises a roller unit or a conveyor belt or straps of the known type wound around cylinders 7 so as to form a ring, wherein at least one of said cylinders is mechanically connected to first power means **8** of the type known per se and essentially comprising a gear motor.

Regarding the sorting station **10,** it can be observed also in the detail Figures 4a and 5a that it is defined along the feeding line **3,** in the area where the outlet and inlet ends of two consecutive ones of said transport surfaces **4** and the inlet end of the control surface **6** converge.

With particular reference to the detail Figures 4a, 5a, in each sorting station **10** it is possible to identify an upstream area **10a** in which there is the outlet end of one of said transport surfaces **4** and a downstream area **10b** in which there are the inlet end of another transport surface **4** adjacent to the previous one and the inlet end of the control surface **6** of the corresponding control station **5.** For the sake of description clarity, at the level of each sorting station **10** and with reference to the direction of advance of the hides indicated by the arrow **F,** it is convenient to identify:
- the transport surface present in the upstream area **10a** of the sorting station **10** as first transport surface **4';**
- the outlet end of the first transport surface **4'** by **4'a;**
- the transport surface present in the downstream area **10b** of the sorting station **10** as second transport surface **4";**
- the inlet end of the second transport surface **4"** by **4"a;**
- the inlet end of the control surface **6** of the corresponding control station **5 by 6a.**

It is also possible to identify second power means **9** that are associated with said surfaces **4** and **6** so as to mutually move them and place the outlet end **4'a** of the first transport surface **4'** in communication with the inlet end **4"a** of the second transport surface **4"** or with the inlet end **6a** of the control surface **6** as is better explained below.

In particular, different mutual configurations of the surfaces **4', 4"** and **6** shown in Figures from 6 to 9 can be obtained by properly operating the second power means **9,** each one of which also in this case comprises at least one gear motor.

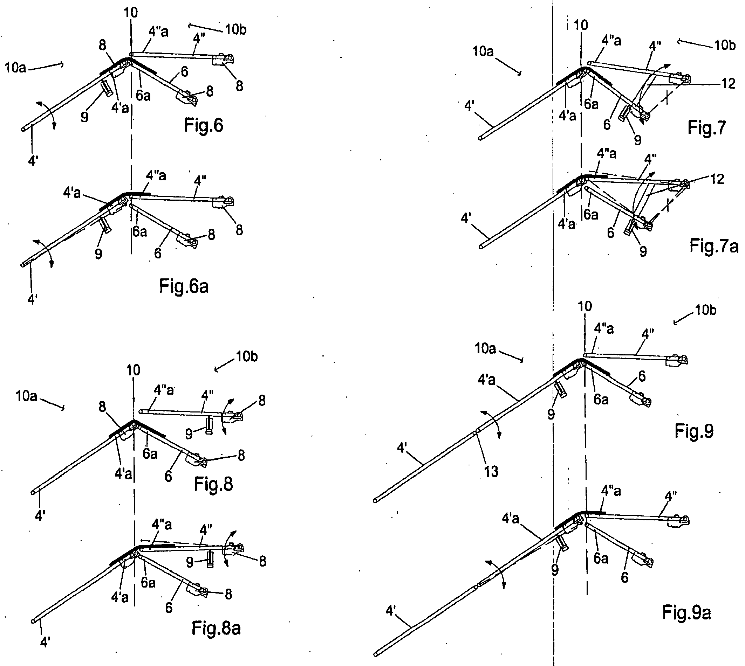

Figures 6 and 6a show an embodiment in which the second power means **9** are associated with the first transport surface **4'** and make it possible to obtain:
- a first operating position shown in Figure 6, in which the outlet end **4'a** of the first transport surface **4'** is near the inlet end **6a** of the control surface **6** belonging to the control station **5** in order to transfer the hide **P** from the first transport surface **4'** to the control surface **6** and then to the control station **5, and**
- a second operating position shown in Figure 6a, in which the outlet end **4'a** of the first transport surface **4'** is near the inlet end **4"a** of the second transport surface **4"** in order to transfer the hide **P** from the first transport surface **4'** to the second transport surface **4".**

Another embodiment of the invention is shown in Figures 8 and 8a, in which it is possible to observe that the second power means **9** are associated with the second transport surface **4" and** make it possible to obtain:
- a first operating position shown in Figure 8, in which the inlet end **4"a** of the second transport surface **4"** is spaced from the outlet end **4'a** of the first transport surface **4'** in order to allow the hide **P** to be transferred from the first transport surface **4'** to the control surface **6** and then to the control station **5, and**
- a second operating position shown in Figure 8a, in which the inlet end **4"a** of the second transport surface **4"** is near the outlet end **4'a** of the first transport surface **4'** in order to allow the hide **P** to be transferred from the first transport surface **4'** to the second transport surface **4".**

A further embodiment of the invention is shown in Figures 7 and 7a, in which it is possible to observe that the second power means **9** are associated with a connection rod **12** that rigidly connects the second transport surface **4"** and the control surface **6** to each other, and make it possible to obtain:
- a first operating position shown in Figure 7, in which the inlet end **6a** of the control surface **6** is near the outlet end **4'a** of the first transport surface **4'** in order to transfer the hide **P** from the first transport surface **4'** to the control surface **6** and then to the control station **5, and**
- a second operating position shown in Figure 7a, in which the inlet end **4"a** of the second transport surface **4"** is near the outlet end **4'a** of the first transport surface 4' in order to transfer the hide **P** from the first transport surface **4'** to the second transport surface **4".**

For all the embodiments illustrated and described with reference to Figures from 6 to 8, a construction variant shown in Figures 9 and 9a is possible, which in the represented version reproduces the configuration of the corresponding Figures 6 and 6a.

It can be observed that according to this variant there is a hinge **13** that makes the outlet end **4'a** of the corresponding first transport surface **4'** movable.

In this construction variant the second power means **9** are connected to the outlet end **4'a** of the first transport surface **4'** and move it with a caliper movement whose fulcrum is the hinge **13,** in order to arrange it in the configurations shown in Figures 9 and 9a.

This construction variant can obviously be applied to any one of the transport and control surfaces of the embodiments illustrated and described with reference to Figures from 6 to 8 and offers the advantage that it can achieve all the mutual positions between the transport surfaces **4'** and **4"** and the control surface **6** described above, by moving only their inlet or outlet ends instead of the entire surface.

Along the feeding line **3** there are automatic means suited to measure the physical characteristics of the hides **P,** like for example means **20** suited to measure the surface area, means **21** suited to weigh the hide, means **22** suited to measure the thickness and similar means.

Furthermore, the operator **O** who checks the hide **P** that moves forward, spread on the control surface **6,** can use a manual unit **23** by means of which, once having examined each hide **P** and based on his/her own experience, he/she assigns the hide **P** a quality code selected among a predefined series of codes.

The automatic measuring means **20, 21, 22** and the manual unit **23** are connected to a classification unit **25** that can be autonomous or integrated in the system's power supply and control panel, which comprises electronic processing and control means suited to classify the quality of the hides by processing the data they receive from the automatic measuring means and by the manual unit **23** through a predefined algorithm.

The result of the data processing operation gives the overall quality classification of each hide and indicates the corresponding pallet or accumulation station where it must be placed and where further hides with the same quality classification will be stacked.

Here below the terms accumulation station and pallet are used as synonyms. From an operational point of view, with reference to Figures 4 and 5, the hides **P** to be classified according to their quality are spread on the feeding line **3** and they advance towards the sorting station **10** passing under the surface area measuring means **20,** the weighing means **21** and the thickness measuring means **22** that transmit the taken data to the classification unit **25.**

If the sorting station **10** is arranged in the configuration that can be observed in Figures 4 and 4a, in which the outlet end **4'a** of the first transport surface **4'** is near the inlet end **6a** of the control surface **6** of the first control station **5',** the first hide **P'** reaches the first operator **O'** who checks it.

Once the check has been completed, the first operator **O'** enters the quality code that he/she decides to assign to the first hide checked **P'** into the manual unit **23,** thus allowing the classification unit **25** to establish the correct quality classification for the same hide.

Downstream of the first control station **5'** a first unloading device **S'** collects the first hide **P'** and places it in the accumulation station constituted, for example, by a pallet **26** that successively will accommodate all the other classified hides that are assigned the same quality code by the classification unit **25.**

Obviously, the first unloading device **S'** has at its disposal several accumulation stations, namely several pallets, divided by quality class, of which only the pallet **26** is shown in the drawing of Figure 4 for the sake of clarity.

In the meantime, the second power means **9,** following a command received, for example, from the control panel (not illustrated herein), switch the configuration of the sorting station **10** and arrange the latter in the configuration shown in Figures 5 and 5a, in which the outlet end **4'a** and the inlet end **4"a** respectively of the first transport surface **4'** and of the second transport surface **4"** are near each other.

In this way the second hide **P"** continues its movement along the feeding line **3** until reaching the control surface **6** of the second control station **5"** where, analogously to that which has already been described, the second hide P" is checked by the second operator **O"** and then collected by the second unloading device **S"** and arranged on a corresponding pallet, not illustrated herein.

The cycle continues with the repositioning of the sorting station **10** in the configuration shown in Figures 4 and 4a, and so on, until all the hides have been checked.

By properly programming the operation of the second power means **9,** it is possible to switch the sorting station **10** according to predefined sequences.

It is thus possible, for example, to program operation so that the hides to be checked are continuously transferred alternately to the first and to the second control station, respectively **5'** and **5".**

It is also possible to program switching sequences of the sorting station **10** of any type, according to the manner in which the hides are to be transferred to the control stations **5'** and **5".**

Finally, the switching sequences of the sorting station **10** can also be selected and managed manually by the operator, based on the needs in terms of type of check and type of hides.

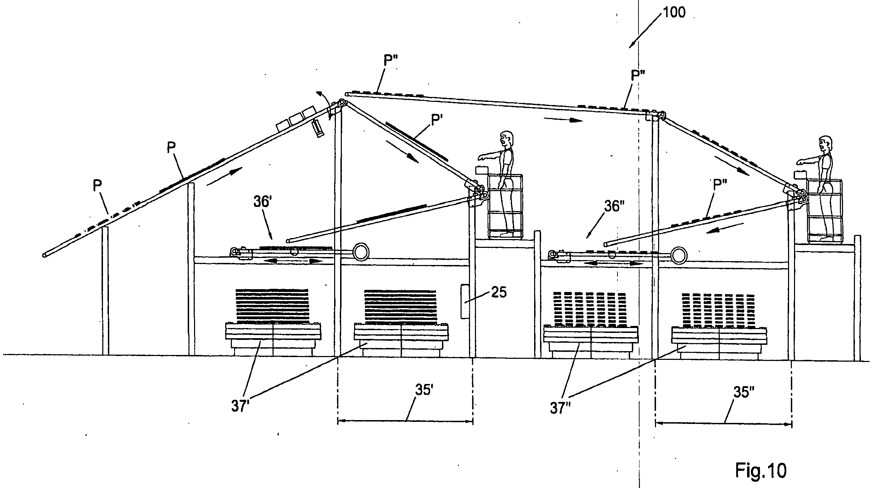

A variant embodiment of the system of the invention is illustrated in Figure 10, where it is indicated as a whole by **100,** and differs from the embodiment previously described with reference to Figures 4 and 5 in that, downstream of the control stations **35'** and **35",** there are transfer means comprising translation carriages indicated by **36'** and **36"** that move the outbound hides towards a plurality of pallets or accumulation stations **37', 37",** each one of which is configured in such a way as to accommodate hides of the same quality.

In this case the hides are accumulated automatically, as the transfer means **36'** and **36"** are controlled directly by the classification unit **25** that chooses the accumulation station where the hides must be transferred based on their quality classification.

It is important to note that the number and the configuration of the transfer means and of the accumulation stations may be different from those illustrated and described in the figures.

So, for example, a different embodiment may include a single translation carriage arranged downstream of both the control stations **35'** and **35"** that distributes the checked hides to both the accumulation stations **37', 37".**

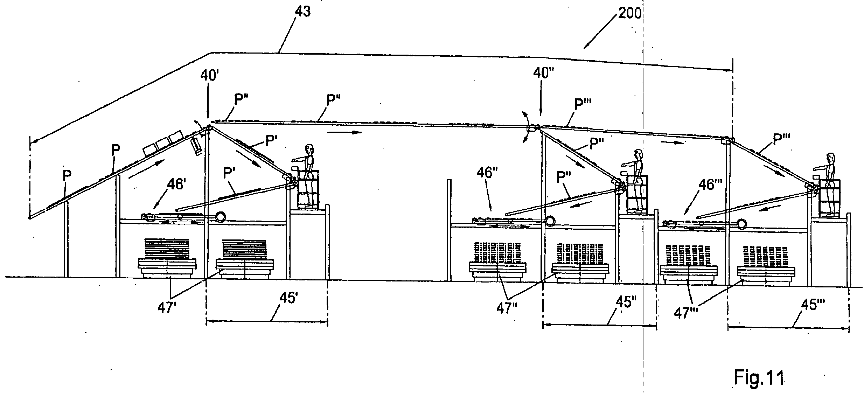

A further variant embodiment of the system of the invention is shown in Figure 11, where it is indicated by **200,** and differs from the embodiments described up to now in that it comprises a feeding line **43** for feeding the hides **P,** in which there are two sorting stations **40'** and **40"** and three control stations **45', 45"** and **45"'** connected in parallel to the feeding line **43.** Furthermore, each control station **45', 45"** and **45"'** is followed by corresponding transfer means **46', 46"** and **46"'** suited to transfer the hides to corresponding accumulation stations **47', 47"** and **47"'.**

Obviously, other variant embodiments with any number of control stations are possible.

According to the description provided above, it can be understood that the system according to the invention achieves all the set objects.

In fact, by arranging any number of control stations in parallel with respect to the hide feeding line, several operators can check simultaneously all the hides entering the feeding line.

In this way, it will be possible to guarantee that the hides coming from the production departments are controlled independently of their quantity, since by distributing them to several control stations it is possible to give each operator sufficient time to perform a careful and effective check on each hide.

In other words, by dividing the total output of hides to be checked among several control stations it is possible to maintain a constant workload for each operator, allowing him/her to perform a standard quality control.

Furthermore, the possibility to program the switching sequence of the sorting stations allows the user to program the duration of the check for each hide in the respective control station.

It will therefore be easier to carry out customized controls on hides with special characteristics.

Furthermore, each sorting station can be arranged according to configurations that make it possible to selectively convey the hides towards just one or more control stations, at the user's discretion.

Obviously, it will also be possible to exclude one or more control stations and have the system run as a system of the known type provided with just one control station.

During construction, the system according to the invention can be subjected to modifications or variants that are neither described herein not illustrated in the drawings.

All these variants or modifications must be considered protected by the present patent, provided that they fall within the scope of the following claims.

## Claims

1. System (1; 100; 200) for classifying hides (P; **P'**, P") according to quality, comprising a frame (2) that supports a feeding line (3; 43) for feeding said hides and a classification unit (25) suited to classify said hides and comprising electronic processing and control means operatively connected to:
- automatic measuring means (20, 21, 22) suited to measure the physical characteristics of said hides and arranged along said feeding line (3; 43), and
- a manual unit (23) suited to assign a quality code chosen by the operator to each one of said hides,
**characterized in that** said feeding line (3; 43) for feeding said hides comprises a plurality of distinct transport surfaces (4; 4', 4") suited to move said hides (P; P', P") forward according to an advance direction (F) towards a plurality of control stations (5; 5', 5") each one of which comprises a control surface (6) for said hides, said control stations (5; 5', 5") being connected in parallel to said feeding line (3; 43) through sorting stations (10; 40', 40") that are configured so as to selectively transfer said hides between said transport surfaces (4; 4', 4") and the control surface (6) belonging to a corresponding one of said control stations (5; 5', 5"), said system comprising first power means (8) suited to move said surfaces (4; 4', 4"; 6) forward and second power means (9) suited to mutually move said surfaces (4; 4', 4"; 6), said power means being suited to allow said hides (P; P', P") to be selectively transferred between said transport surfaces (4; 4', 4") and the control surface (6) that belongs to a corresponding one of said control stations (5; 5'; 5").

2. System (1; 100; 200) according to claim 1, **characterized in that** each one of said sorting stations (10; 40'; 40") is defined along said feeding line (3; 43) in the area where the inlet and outlet ends (4'a; 4"a) of two consecutive ones of said transport surfaces (4'; 4") and the inlet end (6a) of said control surface (6) converge, at least one of said surfaces being associated with said second power means (9).

3. System (1; 100; 200) according to claim 2, **characterized in that** with reference to said advance direction (F) of said hides along said feeding line (3; 43), in each one of said sorting stations (10; 40'; 40") it is possible to identify:
- an upstream area (10a) in which there is the outlet end (4'a) of a first one of said transport surfaces (4');
- a downstream area (10b) in which there are the inlet end (4"a) of a second one of said transport surfaces (4") and the inlet end (6a) of one of said control surfaces (6) belonging to a corresponding control station (5; 5'; 5"),
at least one of said surfaces (4; 4'; 4"; 6) being associated with said second power means (9) that are configured so as to place said outlet end (4'a) of said first transport surface (4') in communication with said inlet end (4"a) of said second transport surface (4") or with said inlet end (6a) of said control surface (6).

4. System (1; 100; 200) according to claim 3, **characterized in that** said second power means (9) are associated with said first transport surface (4') and define two operating positions that comprise:
- a first operating position, in which said outlet end (4'a) of said first transport surface (4') is near said inlet end (6a) of said control surface (6) of said control station (5; 5', 5") in order to transfer the hide from said first transport surface (4') to said control surface (6) of said control station (5; 5', 5"), **and**
- a second operating position, in which said outlet end (4'a) of said first transport surface (4') is near said inlet end (4"a) of said second transport surface (4") in order to transfer the hide from said first transport surface (4') to said second transport surface (4").

5. System (1; 100; 200) according to claim 3, **characterized in that** said second power means (9) are associated with said second transport surface (4") and define two operating positions that comprise:
- a first operating position, in which said inlet end (4"a) of said second transport surface (4") is spaced from said outlet end (4'a) of said first transport surface (4') in order to allow the hide to be transferred from said first transport surface (4') to said control surface (6) and then to said control station (5; 5', 5"), **and**
- a second operating position, in which said inlet end (4"a) of said second transport surface (4") is near said outlet end (4'a) of said first transport surface (4'), in order to allow the hide to be transferred from said first transport surface (4') to said second transport surface (4").

6. System (1; 100; 200) according to claim 3, **characterized in that** said second power means (9) are associated with connection means (12) that rigidly join said second transport surface (4") and said control surface (6) to each other and define:
- a first operating position, in which said inlet end (6a) of said control surface (6) is near said outlet end (4'a) of said first transport surface (4') in order to allow the hide to be transferred from said first transport surface (4') to said control surface (6) and then to said control station (5; 5', 5"), **and**
- a second operating position, in which said inlet end (4"a) of said second transport surface (4") is near said outlet end (4'a) of said first transport surface (4') for the transfer of the hide from the first transport surface (4') to the second transport surface (4").

7. System (1; 100; 200) according to any of the preceding claims, **characterized in that** each one of said surfaces (4; 4'; 4"; 6) comprises a conveyor belt wound as a ring on supporting cylinders (7) mechanically connected to said first power means (8).

8. System (1; 100; 200) according to any of the claims from 1 to 6, **characterized in that** each one of said surfaces (4; 4'; 4"; 6) comprises conveyor belts wound as rings on supporting cylinders (7) mechanically connected to said first power means (8).

9. System (100; 200) according to any of the preceding claims, **characterized in that** it comprises:
- a plurality of hide accumulation stations (37', 37"; 47', 47", 47"') arranged downstream of said control stations (5; 5', 5"), each one of said accumulation stations being configured so that it accommodates hides of the same quality;
- transfer means (36', 36"; 46', 46", 46"') suited to transfer the outbound hides leaving any of said control stations (5; 5', 5") towards a predefined accumulation station (37', 37"; 47', 47", 47"').

## Patentansprüche

1. System (1; 100; 200) zur qualitativen Klassifizierung von Häuten (P; P', P"), einen Rahmen (2) umfassend, der eine Zuführlinie (3; 43) zur Zuführung der besagten Häute trägt sowie eine Klassifizierungseinheit (25), die dazu geeignet ist, die besagten Häute zu klassifizieren und die elektronische Verarbeitungs- und Kontrollmittel umfasst, welche operativ verbunden sind mit:
- automatischen, entlang der besagten Zuführlinie (3; 43) angeordneten Messmitteln (20, 21, 22), dazu geeignet, die physikalischen Merkmale der besagten Häute zu messen, und
- einer manuellen Einheit (23), dazu geeignet, jeder der besagten Häute einen vom Bediener bestimmten Qualitätscode zuzuweisen,
**dadurch gekennzeichnet, dass** die besagte Zuführlinie (3; 43) für die Zuführung der besagten Häute eine Vielzahl einzelner Transportflächen (4; 4', 4") umfasst, die dazu geeignet sind, die besagten Häute (P; P', P") gemäß einer Vorschubrichtung (F) zu einer Vielzahl von Kontrollstationen (5; 5', 5") vorwärts zu bewegen, von denen jede eine Kontrollfläche (6) für die besagten Häute umfasst, und wobei die besagten Kontrollstationen (5; 5', 5") parallel mit der besagten Zuführlinie (3; 43) verbunden sind über Sortierstationen (10; 40', 40"), die derart konfiguriert sind, dass sie die besagten Häute selektiv zwischen den besagten Transportflächen (4; 4', 4") und der zu einer entsprechenden der besagten Kontrollstationen (5; 5', 5") gehörenden Kontrollfläche (6) transferieren, wobei das besagte System erste Antriebsmittel (8) umfasst, die geeignet sind, die besagten Flächen (4; 4', 4"; 6) vorwärts zu bewegen, sowie zweite Antriebsmittel (9), die geeignet sind, die besagten Flächen (4; 4', 4"; 6) wechselseitig zu bewegen, wobei die besagten Antriebsmittel geeignet sind, zu erlauben, dass die besagten Häute (P; P', P") selektiv zwischen den besagten Transportflächen (4; 4', 4") und der zu einer entsprechenden der besagten Kontrollstationen (5; 5'; 5") gehörenden Kontrollfläche (6) transferiert werden.

2. System (1; 100; 200) gemäß Patentanspruch 1, **dadurch gekennzeichnet, dass** jede der besagten Sortierstationen (10; 40'; 40") entlang der besagten Zuführlinie (3; 43) definiert ist, in dem Bereich, in dem die Ein- und Ausgangsenden (4'a; 4"a) von zwei aufeinanderfolgenden der besagten Transportflächen (4'; 4") und das Eingangsende (6a) der besagten Kontrollfläche (6) konvergieren, wobei wenigstens eine der besagten Flächen mit den besagten Antriebsmitteln (9) verbunden ist.

3. System (1; 100; 200) gemäß Patentanspruch 2, **dadurch gekennzeichnet, dass** es bezüglich der besagten Vorschubrichtung (F) der besagten Häute entlang der besagten Zuführlinie (3; 43) in jeder der besagten Sortierstationen (10; 40'; 40") möglich ist, Folgendes zu identifizieren:
- einen vorgeschalteten Bereich (10a), in dem sich das Ausgangsende (4'a) einer ersten der besagten Transportflächen (4') befindet;
- einen nachgeschalteten Bereich (10b), in dem sich das Eingangsende (4"a) einer zweiten der besagten Transportflächen (4") befindet sowie das Eingangsende (6a) einer der besagten, zu einer entsprechenden Kontrollstation (5; 5'; 5") gehörenden Kontrollflächen (6),
wobei wenigstens eine der besagten Flächen (4; 4'; 4"; 6) mit den besagten, zweiten Antriebsmitteln (9) verbunden ist, welche derart konfiguriert sind, dass sie das besagte Ausgangsende (4'a) der besagten, ersten Transportfläche (4') mit dem besagten Eingangsende (4"a) der besagten, zweiten Transportfläche (4") oder mit dem besagten Eingangsende (6a) der besagten Kontrollfläche (6) in Verbindung setzen.

4. System (1; 100; 200) gemäß Patentanspruch 3, **dadurch gekennzeichnet, dass** die besagten, zweiten Antriebsmittel (9) mit der besagten, ersten Transportfläche (4') verbunden sind und zwei Arbeitspositionen definieren, die Folgendes umfassen:
- eine erste Arbeitsposition, in der das besagte Ausgangsende (4'a) der besagten, ersten Transportfläche (4') sich nahe am besagten Eingangsende (6a) der besagten Kontrollfläche (6) der besagten Kontrollstation (5; 5', 5") befindet, um die Haut von der besagten, ersten Transportfläche (4') zu der besagten Kontrollfläche (6) der besagten Kontrollstation (5; 5', 5") zu transferieren,
und
- eine zweite Arbeitsposition, in der das besagte Ausgangsende (4'a) der besagten, ersten Transportfläche (4') sich nahe am besagten Eingangsende (4"a) der besagten, zweiten Transportfläche (4") befindet, um die Haut von der besagten, ersten Transportfläche (4') zu der besagten, zweiten Transportfläche (4") zu transferieren.

5. System (1; 100; 200) gemäß Patentanspruch 3, **dadurch gekennzeichnet, dass** die besagten, zweiten Antriebsmittel (9) mit der besagten, zweiten Transportfläche (4") verbunden sind und zwei Arbeitspositionen definieren, die Folgendes umfassen:
- eine erste Arbeitsposition, in der das besagte Eingangsende (4"a) der besagten, zweiten Transportfläche (4") von dem besagten Ausgangsende (4'a) der besagten, ersten Transportfläche (4') distanziert ist, damit die Haut von der besagten, ersten Transportfläche (4') zu der besagten Kontrollfläche (6) und danach zu der besagten Kontrollstation (5; 5', 5") transferiert werden kann,
und
- eine zweite Arbeitsposition, in der das besagte Eingangsende (4"a) der besagten, zweiten Transportfläche (4") sich nahe am besagten Ausgangsende (4'a) der besagten, ersten Transportfläche (4') befindet, damit die Haut von der besagten, ersten Transportfläche (4') zu der besagten, zweiten Transportfläche (4") transferiert werden kann.

6. System (1; 100; 200) gemäß Patentanspruch 3, **dadurch gekennzeichnet, dass** die besagten, zweiten Antriebsmittel (9) mit Verbindungsmitteln (12) verbunden sind, welche die besagte, zweite Transportfläche (4") und die besagte Kontrollfläche (6) starr miteinander verbinden und Folgendes definieren:
- eine erste Arbeitsposition, in der sich das besagte Eingangsende (6a) der besagten Kontrollfläche (6) nahe an dem besagten Ausgangsende (4'a) der besagten, ersten Transportfläche (4') befindet, damit die Haut von der besagten, ersten Transportfläche (4') zu der besagten Kontrollfläche (6) und danach zu der besagten Kontrollstation (5; 5', 5") transferiert werden kann,
und
- eine zweite Arbeitsposition, in der das besagte Eingangsende (4"a) der besagten, zweiten Transportfläche (4") sich nahe am besagten Ausgangsende (4'a) der besagten, ersten Transportfläche (4') befindet, um die Haut von der besagten, ersten Transportfläche (4') zu der zweiten Transportfläche (4") zu transferieren.

7. System (1; 100; 200) gemäß eines jeden der vorstehenden Patentansprüche, **dadurch gekennzeichnet, dass** jede der besagten Flächen (4; 4'; 4"; 6) ein Förderband umfasst, das wie ein Ring um Tragzylinder (7) gewickelt ist, welche mechanisch mit den besagten, ersten Antriebsmitteln (8) verbunden sind.

8. System (1; 100; 200) gemäß eines jeden der Patentansprüche von 1 bis 6, **dadurch gekennzeichnet, dass** jede der besagten Flächen (4; 4'; 4"; 6) Förderbänder umfasst, die wie Ringe um Tragzylinder (7) gewickelt sind, welche mechanisch mit den besagten, ersten Antriebsmitteln (8) verbunden sind.

9. System (100; 200) gemäß eines jeden der vorstehenden Patentansprüche, **dadurch gekennzeichnet, dass** es Folgendes umfasst:
- eine Vielzahl von stromabwärts der besagten Kontrollstationen (5; 5', 5") angeordneten Hautsammelstationen (37', 37"; 47', 47", 47"'), wobei jede der besagten Sammelstationen so konfiguriert ist, dass sie Häute derselben Qualität aufnehmen kann;
- Transfermittel (36', 36"; 46', 46", 46"'), dazu geeignet, die aus einer jeglichen der besagten Kontrollstationen (5; 5', 5") auslaufenden Häute zu einer vorbestimmten Sammelstation (37', 37"; 47', 47", 47'") zu transferieren.

## Revendications

1. Système (1; 100; 200) pour la classification de qualité de peaux (P; P', P"), comprenant un châssis (2) qui supporte une ligne d'alimentation (3; 43) desdites peaux et une unité de classification (25) apte à classifier lesdites peaux et qui comprend des moyens électroniques d'élaboration et de contrôle opérationnellement reliés à:
- des moyens automatiques de mesure (20, 21, 22) aptes à mesurer les caractéristiques physiques desdites peaux et disposés le long de ladite ligne d'alimentation (3; 43), et
- une unité manuelle (23) indiquée pour attribuer à chacune desdites peaux un code de qualité choisi par l'opérateur,
**caractérisé en ce que** ladite ligne d'alimentation (3; 43) pour alimenter lesdites peaux comprend une pluralité de surfaces de transport (4; 4', 4") distinguées entre elles, indiquées pour l'avance desdites peaux (P; P', P") selon une direction d'avance (F) vers une pluralité de stations de contrôle (5; 5', 5") chacune desquelles comprend une surface de contrôle (6) pour lesdites peaux, lesdites stations de contrôle (5; 5', 5") étant reliées en parallèle à ladite ligne d'alimentation (3; 43) au moyen de stations de triage (10; 40', 40") qui sont configurées pour transférer sélectivement lesdites peaux entre lesdites surfaces de transport (4; 4', 4") et la surface de contrôle (6) appartenant à une station correspondante desdites stations de contrôle (5; 5', 5"), ledit système comprenant de premiers moyens de motorisation (8) indiqués pour l'avance desdites surfaces (4; 4', 4"; 6) et de deuxièmes moyens de motorisation (9) indiqués pour le déplacement réciproque desdites surfaces (4; 4', 4"; 6), lesdits moyens de motorisation étant aptes à consentir ledit transfert sélectif desdites peaux (P; P', P") entre lesdites surfaces de transport (4; 4', 4") et la surface de contrôle (6) qui appartient à une station correspondante desdites stations de contrôle (5; 5'; 5").

2. Système (1; 100; 200) selon la revendication 1, **caractérisé en ce que** chacune desdites stations de triage (10; 40'; 40") est définie le long de ladite ligne d'alimentation (3; 43) dans la zone où les extrémités de sortie et d'entrée (4'a; 4"a) de deux desdites surfaces de transport (4'; 4") consécutives et l'extrémité d'entrée (6a) de ladite surface de contrôle (6) convergent entre elles, au moins une desdites surfaces étant associée auxdits deuxièmes moyens de motorisation (9).

3. Système (1; 100; 200) selon la revendication 2, **caractérisé en ce qu**'en se référant à ladite direction d'avance (F) desdites peaux le long de ladite ligne d'alimentation (3; 43), en chacune desdites stations de triage (10; 40'; 40") il est possible d'identifier:
- une zone en amont (10a) où se trouve l'extrémité de sortie (4'a) d'une première desdites surfaces de transport (4');
- une zone en aval (10b) où sont présentes l'extrémité d'entrée (4"a) d'une deuxième desdites surfaces de transport (4") et l'extrémité d'entrée (6a) d'une desdites surfaces de contrôle (6) appartenant à une station de contrôle correspondante (5; 5'; 5"),
au moins une desdites surfaces (4; 4'; 4"; 6) étant associés auxdits deuxièmes moyens de motorisation (9) qui sont configurés pour mettre en communication ladite extrémité de sortie (4'a) de ladite première surface de transport (4') avec ladite extrémité d'entrée (4"a) de ladite deuxième surface de transport (4") ou avec ladite extrémité d'entrée (6a) de ladite surface de contrôle (6).

4. Système (1; 100; 200) selon la revendication 3, **caractérisé en ce que** lesdits deuxièmes moyens de motorisation (9) sont associés à ladite première surface de transport (4') et définissent deux positions opérationnelles qui comprennent:
- une première position opérationnelle où ladite extrémité de sortie (4'a) de ladite première surface de transport (4') est rapprochée de ladite extrémité d'entrée (6a) de ladite surface de contrôle (6) de ladite station de contrôle (5; 5', 5") de façon à transférer la peau de ladite première surface de transport (4') à ladite surface de contrôle (6) de ladite station de contrôle (5; 5', 5"),
et
- une deuxième position opérationnelle où ladite extrémité de sortie (4'a) de ladite première surface de transport (4') est rapprochée de ladite extrémité d'entrée (4"a) de ladite deuxième surface de transport (4") de façon à transférer la peau de ladite première surface de transport (4') à ladite deuxième surface de transport (4").

5. Système (1; 100; 200) selon la revendication 3, **caractérisé en ce que** lesdits deuxièmes moyens de motorisation (9) sont associés à ladite deuxième surface de transport (4") et définissent deux positions opérationnelles qui comprennent:
- une première position opérationnelle où ladite extrémité d'entrée (4"a) de ladite deuxième surface de transport (4") est espacée de ladite extrémité de sortie (4'a) de ladite première surface de transport (4') de façon à consentir le transfert de la peau de ladite première surface de transport (4') à ladite surface de contrôle (6) et ensuite à ladite station de contrôle (5; 5', 5"),
et
- une deuxième position opérationnelle où ladite extrémité d'entrée (4"a) de ladite deuxième surface de transport (4") est rapprochée de ladite extrémité de sortie (4'a) de ladite première surface de transport (4') de façon à consentir le transfert de la peau de ladite première surface de transport (4') à ladite deuxième surface de transport (4").

6. Système (1; 100; 200) selon la revendication 3, **caractérisé en ce que** lesdits deuxièmes moyens de motorisation (9) sont associés à des moyens de raccordement (12) qui unissent rigidement entre elles ladite deuxième surface de transport (4") et ladite surface de contrôle (6) et définissent:
- une première position opérationnelle où ladite extrémité d'entrée (6a) de ladite surface de contrôle (6) est rapprochée de ladite extrémité de sortie (4'a) de ladite première surface de transport (4') de façon à consentir le transfert de la peau de ladite première surface de transport (4') à ladite surface de contrôle (6) et ensuite à ladite station de contrôle (5; 5', 5"),
et
- une deuxième position opérationnelle où ladite extrémité d'entrée (4"a) de ladite deuxième surface de transport (4") est rapprochée de ladite extrémité de sortie (4'a) de ladite première surface de transport (4') pour le transfert de la peau de la première surface de transport (4') à la deuxième surface de transport (4").

7. Système (1; 100; 200) selon l'une quelconque des revendications précédentes, **caractérisé en ce que** chacune desdites surfaces (4; 4'; 4"; 6) comprend une courroie transporteuse enroulée en anneau sur des cylindres de support (7) mécaniquement reliés auxdits premiers moyens de motorisation (8).

8. Système (1; 100; 200) selon l'une quelconque des revendications de 1 à 6, **caractérisé en ce que** chacune desdites surfaces (4; 4'; 4"; 6) comprend des courroies transporteuses enroulées en anneaux sur des cylindres de support (7) mécaniquement reliés auxdits premiers moyens de motorisation (8).

9. Système (100; 200) selon l'une quelconque des revendications précédentes, **caractérisé en ce qu**'il comprend:
- une pluralité de stations d'accumulation de peaux (37', 37"; 47', 47", 47"') disposées en aval desdites stations de contrôle (5; 5', 5"), chacune desdites stations d'accumulation étant configurée de façon à accueillir des peaux de la même qualité;
- des moyens de transfert (36', 36"; 46', 46", 46"') indiqués pour transférer les peaux en sortie d'une quelconque desdites stations de contrôle (5; 5', 5") vers une station d'accumulation préétablie (37', 37"; 47', 47", 47"').
